Europäisches Patentamt

European Patent Office

Office européen des brevets

(11) Veröffentlichungsnummer: **0 505 746 A2**

## EUROPÄISCHE PATENTANMELDUNG

(21) Anmeldenummer: **92103127.4**

(22) Anmeldetag: **25.02.92**

(51) Int. Cl.5: **C07C 53/10**, C07C 51/41

(30) Priorität: **26.03.91 DE 4109821**

(43) Veröffentlichungstag der Anmeldung:
**30.09.92 Patentblatt 92/40**

(84) Benannte Vertragsstaaten:
**AT BE CH DE DK FR GB IT LI NL SE**

(71) Anmelder: **HOECHST AKTIENGESELLSCHAFT**
**Postfach 80 03 20**
**W-6230 Frankfurt am Main 80(DE)**

(72) Erfinder: **Bettermann, Gerhard, Dr.**
**Alfons-Keever-Strasse 54**
**W-5164 Nörvenich-Pingsheim(DE)**
Erfinder: **Schimmel, Günther, Dr.**
**Ehrenstrasse 16**
**W-5042 Erftstadt(DE)**

(54) **Verfahren zur Herstellung von Erdalkaliacetaten.**

(57) Zur Herstellung von Erdalkaliacetaten durch Umsetzung der entsprechenden Erdalkalioxide, -hydroxide oder -carbonate mit 85 bis 100 %iger Essigsäure gibt man mindestens eine der Calciumverbindungen und/oder mindestens eine der Magnesiumverbindungen mit einem stöchiometrischen Überschuß von bis zu 1,9 % an Essigsäure am Anfang einer Mischstrecke auf und bewegt das Material darin während 10 bis 100 Minuten unter dauerndem Mischen fort. An einem Punkt der Mischstrecke, welchen die Mischung nach frühestens 5 Minuten erreicht, setzt man bis zu 1,9 Mol% einer basisch wirkenden Alkaliverbindung, bezogen auf die aufgegebene Essigsäure, zu. Dabei weist eine 10 %ige wäßrige Lösung des die Mischstrecke verlassenden Reaktionsproduktes einen pH-Wert von 7,5 bis 10 auf.

EP 0 505 746 A2

Die vorliegende Erfindung betrifft ein Verfahren zur Herstellung von Erdalkaliacetaten durch Umsetzung der entsprechenden Erdalkalioxide, -hydroxide oder -carbonate mit 85 bis 100 %iger Essigsäure.

Die Verwendung von Chloriden des Natriums, Calciums oder Magnesiums in Winterdienst für Straßen und Fahrbahnen ist wegen der durch sie verursachten Korrosionsschäden und ökologischen Beeinträchtigungen so nachteilig, daß in den USA bereits vor einiger Zeit vorgeschlagen wurde, Calcium-Magnesium-Acetat (CMA) als Tausalz wegen seiner guten Wirksamkeit, seiner antikorrosiven Eigenschaften und seiner geringen Umweltrelevanz zu verwenden.

Ein Verfahren zur Herstellung granulierter Calcium- und Magnesiumacetate ist aus der DE-PS 37 05 618 bekannt. Danach werden Oxide oder Hydroxide von Calcium oder Magnesium mit einem stöchiometrischen Überschuß von 2 bis 10 % an 85 bis 100 %iger Essigsäure in einem Mischreaktor unter Rückflußkühlung umgesetzt, wobei gleichzeitig Granulation erfolgt. Anschließend wird in einem Bewegungsreaktor die restliche Essigsäure und Wasser aus dem Reaktionsprodukt entfernt.

Schließlich wird beim Verfahren zur Herstellung einer Lösung von Calciumacetat mit einem pH-Wert zwischen 7 und 8 (bei Raumtemperatur) nach der US-PS 4 377 488 Essigsäure mit einer solchen Menge von gebranntem Kalkstein umgesetzt, die 95 bis 98 % der stöchiometrischen Menge entspricht. In einem zweiten Schritt wird wäßrige Natronlauge oder Kalilauge zugefügt, wobei deren Menge 2 bis 5 % der stöchiometrischen Menge, bezogen auf die eingesetzte Essigsäure, beträgt.

Bei dem zuerst genannten Verfahren ist von Nachteil, daß die Entfernung der unreagierten Essigsäure aus dem Reaktionsprodukt in aufwendiger Weise durch Trocknen bei 140 bis 150 ° C erfolgt.

Nachteilig ist bei dem zuletzt genannten Verfahren, daß es das Enteisungsmittel in Form einer Lösung bereitstellt, deren Lagerung besondere Einrichtungen erfordert oder eine energieintensive Trocknung der Lösung zu einem festen Produkt nötig macht.

Es ist daher Aufgabe der vorliegenden Erfindung, ein Verfahren zur Herstellung von Erdalkaliacetaten aus Sauerstoffverbindungen von Erdalkalien und Essigsäure anzugeben, bei welchem das Reaktionsprodukt ein Feststoff ist, welcher weitgehend wasserlöslich ist und dessen wäßrige Lösung keine saure Reaktion zeigt. Das wird erfindungsgemäß dadurch erreicht, daß man mindestens eine der Calciumverbindungen und/oder mindestens eine der Magnesiumverbindungen mit einem stöchiometrischen Überschuß von bis zu 1,9 % an Essigsäure am Anfang einer Mischstrecke aufgibt und darin während 10 bis 100 Minuten, vorzugsweise 15 bis 30 Minuten, unter dauerndem Mischen fortbewegt und daß man an einem Punkt der Mischstrecke, welchen die Mischung nach frühestens 5 Minuten erreicht, bis zu 1,9 Mol% einer basisch wirkenden Alkaliverbindung, bezogen auf die aufgegebene Essigsäure, zusetzt, wobei eine 10 %ige wäßrige Lösung des die Mischstrecke verlassenden Reaktionsproduktes einen pH-Wert von 7,5 bis 10, vorzugsweise von 8,2 bis 10, aufweist.

Das Verfahren gemäß der Erfindung kann weiter wahlweise auch noch dadurch ausgestaltet sein, daß

a) man als Calcium- und Magnesiumverbindung gebrannten Dolomit einsetzt;

b) bei Temperaturen von 1100 bis 1200 ° C 60 bis 270 s weich gebrannter Dolomit verwendet wird;

c) man als Calciumverbindung gebrannten Kalkstein einsetzt;

d) man als Magnesiumverbindung weich gebrannten Magnesit einsetzt;

e) bei Temperaturen von 550 bis 650 ° C weich gebrannter Magnesit verwendet wird;

f) als basisch wirkende Alkaliverbindungen wäßrige Lösungen von Natriumhydroxid, Kaliumhydroxid, Natriumcarbonat, Kaliumcarbonat oder deren Mischungen dienen;

g) man das die Mischstrecke verlassende Produkt auf eine Kornfeinheit von 100 bis 800 $\mu$m aufmahlt;

h) man das aufgemahlene Material zu seiner Kompaktierung einer Preßgranulation unterwirft;

i) man das Preßgranulat auf Teilchengrößen zwischen 0,1 und 5 mm, vorzugsweise zwischen 0,5 und 2 mm, zerkleinert.

Unter weichgebranntem Dolomit oder weichgebranntem Magnesit versteht man Magnesium-Calcium-Oxid bzw. Magnesiumoxide, welche unter solchen Bedingungen aus Dolomit bzw. Magnesit gebrannt wurden, daß eine desaktivierende Sinterung unter Bildung wenig reaktiven Dolomits bzw. Magnesits soweit wie möglich unterblieben ist. Dolomit wird dazu bei Temperaturen von 1100 bis 1200 ° C einige Minuten im Drehrohrofen behandelt, während bei niedrigen Temperaturen längere Verweilzeiten erforderlich sind. Für Magnesit gelten Temperaturen von etwa 550 bis 650 ° C. Sog. normal gebrannter Dolomit ist bei höherer Temperatur längere Zeit behandelt worden und ist weniger reaktiv, weil das in ihm enthaltene Magnesiumoxid und andere Beimengungen zumindest teilweise gesintert sind.

Das beim erfindungsgemäßen Verfahren als zerkleinertes Preßgranulat anfallende Reaktionsprodukt ist kantig, so daß es von Fahrbahnen oder Eisflächen nicht abrollt.

Das Reaktionsprodukt des Verfahrens gemäß der Erfindung ist wegen seiner nahezu vollständigen Wasserlöslichkeit gut wirksam, wobei sein Verbleiben an der Applikationsstelle von seiner die Lösegeschwindigkeit bestimmenden Korngröße abhängig ist.

Das beim erfindungsgemäßen Verfahren anfallende Reaktionsprodukt verursacht bei seiner Anwendung als Enteisungsmittel praktisch keine Korrosion.

Das Reaktionsprodukt des Verfahrens gemäß der Erfindung wirkt dem Versauern des Ackerbodens entgegen.

Beispiel 1 (Vergleichsbeispiel)

In einen Intensivmischer (REAKTOTHERM der Fa. Krauss-Maffei) wurden 1 kg weich gebrannter Dolomit (10,4 mol) mit einer Korngröße von 100 % < 500 $\mu$m und 2558 g 99,5 %ige Essigsäure (42,4 mol) eingetragen. Nach einer Gesamtmischzeit von 30 Minuten resultierte ein Reaktionsprodukt mit einem in Wasser unlöslichen Rückstand von 2,4 %, dessen 10 %ige wäßrige Suspension einen pH-Wert von 3,2 aufwies.

Beispiel 2 (Vergleichsbeispiel)

Beispiel 1 wurde mit Änderung wiederholt, daß nach einer Verweilzeit von Dolomit und Essigsäure im Mischer von 5 Minuten 104 g 45 %ige Kalilauge (0,83 mol) hinzugefügt und 25 Minuten weitergemischt wurde. Danach resultierte ein Reaktionsprodukt mit einem in Wasser unlöslichen Rückstand von 9,5 %, dessen 10 %ige wäßrige Suspension einen pH-Wert von 8,9 aufwies.

Beispiel 3 (gemäß der Erfindung)

An dem einen Ende einer etwa 6 m langen Paddelschnecke wurden kontinuierlich 10 kg/h des in Beispiel 1 verwendeten Dolomits (104 mol) mit einer Korngröße von 100 % < 500 $\mu$m und 25,6 kg/h 99,5 %ige Essigsäure (424 mol) eingespeist. Nach einer Verweilzeit von 20 Minuten, während das Gemisch durch die Paddelschnecke hindurchbewegt wurde, wurden etwa 4 m vom Eintragsende der Paddelschnecke entfernt 104 g/h 45 %ige Kalilauge (0,83 mol) in feinverteilter Form hinzugefügt. Nach einer Gesamtverweilzeit in der Paddelschnecke von 30 Minuten wurde an ihrem anderen Ende ein Reaktionsprodukt mit einem in Wasser unlöslichen Rückstand von 2,3 % ausgetragen, dessen 10 %ige wäßrige Lösung einen pH-Wert von 9,2 aufwies.

Beispiel 4 (gemäß der Erfindung)

Das Reaktionsprodukt aus Beispiel 3 wurde auf einer Hammermühle auf eine Kornfeinheit von < 1 mm aufgemahlen.

Das feingemahlene Reaktionsprodukt wurde in einem Preßgranulator (WP 50 der Fa. Alexanderwerk) zu plattenförmigen Teilchen geformt. Diese Teilchen wurden gebrochen und die Kornfraktion von 0,6 bis 1,8 mm ausgesiebt. Das ausgesiebte Material bestand aus kantigen, unregelmäßig gestalteten Partikeln.

Beispiel 5 (gemäß der Erfindung)

Beispiel 3 wurde mit der Änderung wiederholt, daß die Kalilauge durch 67 g/h 50 % Natronlauge (0,83 mol) ersetzt wurde. Das ausgetragene Reaktionsprodukt, dessen 10 %ige wäßrige Lösung einen pH-Wert von 9,1 aufwies, hatte einen in Wasser unlöslichen Rückstand von 2,5 %.

Beispiel 6 (Vergleichsbeispiel)

Beispiel 3 wurde mit der Änderung wiederholt, daß bei 1700 bis 1800°C gebrannter Dolomit verwendet wurde. Das Reaktionsprodukt wies in 10 %iger wäßriger Lösung einen pH-Wert von 2,9 und einen in Wasser unlöslichen Rückstand von 23,7 % auf.

Beispiel 7 (gemäß der Erfindung)

Beispiel 3 wurde mit der Änderung wiederholt, daß anstelle des Dolomits 1,17 kg/h (20,8 mol) CaO eingespeist wurden. Das Reaktionsprodukt wies einen in Wasser unlöslichen Rückstand von 1,9 % auf, seine 10 %ige wäßrige Lösung einen pH-Wert von 9,4.

Beispiel 8 (gemäß der Erfindung)

Beispiel 3 wurde mit der Änderung wiederholt, daß anstelle des Dolomits 0,84 kg/h MgO (20,8 mol) eingespeist wurden. Das Reaktionsprodukt wies einen in Wasser unlöslichen Rückstand von 2,9 % auf, seine 10 %ige wäßrige Lösung einen pH-Wert von 8,3.

**Patentansprüche**

1.  Verfahren zur Herstellung von Erdalkaliacetaten durch Umsetzung der entsprechenden Erdalkalioxide, -hydroxide oder -carbonate mit 85 bis 100 %iger Essigsäure, dadurch gekennzeichnet, daß man mindestens eine der Calciumverbindungen und/oder mindestens eine der Magnesiumverbindungen mit einem stöchiometrischen Überschuß von bis zu 1,9 % an Essigsäure am Anfang einer Mischstrecke aufgibt und darin während 10 bis 100 Minuten, vorzugsweise 15 bis 30 Minuten, unter dauerndem Mischen fortbewegt, und daß man an einem Punkt der Mischstrecke, welchen die Mischung nach frühestens 5 Minuten erreicht, bis zu 1,9 Mol% einer basisch wirkenden Alkaliverbindung, bezogen auf die aufgegebene Essigsäure, zusetzt, wobei eine 10 %ige wäßrige Lösung des die Mischstrecke verlassenden Reaktionsproduktes einen pH-Wert von 7,5 bis 10, vorzugsweise von 8,2 bis 10, aufweist.

2. Verfahren nach Anspruch 1, dadurch gekennzeichnet, daß man als Calcium- und Magnesiumverbindung gebrannten Dolomit einsetzt.

3. Verfahren nach Anspruch 2, dadurch gekennzeichnet, daß bei Temperaturen von 1100 bis 1200°C 60 bis 270 s weich gebrannter Dolomit verwendet wird.

4. Verfahren nach Anspruch 1, dadurch gekennzeichnet, daß man als Calciumverbindung gebrannten Kalkstein einsetzt.

5. Verfahren nach Anspruch 1, dadurch gekennzeichnet, daß man als Magnesiumverbindung weich gebrannten Magnesit einsetzt.

6. Verfahren nach Anspruch 5, dadurch gekennzeichnet, daß bei Temperaturen von 550 bis 650°C weich gebrannter Magnesit verwendet wird.

7. Verfahren nach mindestens einem der Ansprüche 1 bis 6, dadurch gekennzeichnet, daß als basisch wirkende Alkaliverbindungen wäßrige Lösungen von Natriumhydroxid, Kaliumhydroxid, Natriumcarbonat, Kaliumcarbonat oder deren Mischungen dienen.

8. Verfahren nach mindestens einem der Ansprüche 1 bis 7, dadurch gekennzeichnet, daß man das die Mischstrecke verlassende Produkt auf eine Kornfeinheit von 100 bis 800 µm aufmahlt.

9. Verfahren nach Anspruch 8, dadurch gekennzeichnet, daß man das aufgemahlene Material zu seiner Kompaktierung einer Preßgranulation unterwirft.

10. Verfahren nach Anspruch 9, dadurch gekennzeichnet, daß man das Preßgranulat auf Teilchengrößen zwischen 0,1 und 5 mm, vorzugsweise zwischen 0,5 und 2 mm, zerkleinert.